# EUROPEAN PATENT SPECIFICATION

(11) **EP 2 152 302 B1**
(45) Date of publication and mention of the grant of the patent: **07.10.2015**
(21) Application number: 08789070.3
(22) Date of filing: 04.06.2008
(51) Int. Cl.: A61K 39/02, A61K 39/095, A61K 39/116, A61K 39/00, A61K 39/102, A61K 47/48

(54) **FORMULATION OF MENINGITIS VACCINES**
FORMULIERUNG EINES IMPFSTOFFES GEGEN MENINGITIS
FORMULATION DE VACCINS CONTRE LA MÉNINGITE

(30) Priority: 04.06.2007 US 933235 P
(43) Date of publication of application: 17.02.2010
(73) Proprietor: GlaxoSmithKline Biologicals SA, 1330 Rixensart (BE)
(72) Inventor: CONTORNI, Mario, I-53100 Siena (IT); COSTANTINO, Paolo, I-53100 Siena (IT)
(74) Representative: Sampson, Catherine
(86) International application number: PCT/IB2008/002121
(87) International publication number: WO 2008/149238

(56) References cited:
- WO-A-96/14086
- WO-A-03/007985
- WO-A-2005/089794
- WO-A-2007/060548
- WO-A-2009/050586
- AMIR J ET AL: "Immunogenicity and safety of a liquid combination of DT-PRP-T vs lyophilized PRP-T reconstituted with DTP" VACCINE, BUTTERWORTH SCIENTIFIC. GUILDFORD, GB, vol. 15, no. 2, 1 February 1997 (1997-02-01), pages 149-154, XP004054364 ISSN: 0264-410X
- MENDELMAN P M ET AL: "Immunogenicity and safety of Haemophilus influenzae type b polysaccharide-Neisseria meningitidis conjugate vaccine in 7.5 mug liquid formulation: a comparison of three lots with the 15.0 mug lyophilized formulation" VACCINE, BUTTERWORTH SCIENTIFIC. GUILDFORD, GB, vol. 15, no. 6-7, 1 April 1997 (1997-04-01), pages 775-781, XP004064542 ISSN: 0264-410X
- FALUGI F ET AL: "Rationally designed strings of promiscuous CD4(+) T cell epitopes provide help to Haemophilus influenzae type b oligosaccharide: a model for new conjugate vaccines" EUROPEAN JOURNAL OF IMMUNOLOGY, WILEY - V C H VERLAG GMBH & CO. KGAA, DE, vol. 31, no. 12, 1 December 2001 (2001-12-01), pages 3816-3824, XP002401049 ISSN: 0014-2980

## Description

### TECHNICAL FIELD

This invention is in the field of formulating combination vaccines for immunising against meningitis.

### BACKGROUND ART

Vaccines containing antigens from more than one pathogenic organism within a single dose are known as "multivalent" or "combination" vaccines *e.g*. diphtheria, tetanus & pertussis ("DTP") vaccines and measles, mumps & rubella ("MMR") vaccines. Combination vaccines offer patients the advantage of receiving a reduced number of injections, which leads to the clinical advantage of increased compliance (*e.g*. see chapter 29 of reference 1), particularly for pediatric vaccination. At the same time, however, they present difficulties due to factors including: physical and biochemical incompatibility between antigens and other components; immunological interference; and stability.

Some of these difficulties can be addressed by suitable formulation of the vaccine. For instance, the conjugated PRP capsular saccharide of *Haemophilus influenzae* type B ("Hib") can be unstable in aqueous conditions and so Hib-containing vaccines in the INFANRIX™ series (including PEDIARIX™) include a lyophilised Hib component that is re-constituted at the time of use by an aqueous formulation of the remaining antigens. Reference 2 also describes the formulation of Hib-containing vaccines, and the Hib conjugate is lyophilised in combination with meningococcal conjugates, for extemporaneous reconstitution. In contrast, reference 3 describes fully-liquid formulations of meningococcal conjugates, in which further components (e.g. Hib or pneumococcal conjugates) may be lyophilised and reconstituted. Reference 22 describes combinations of meningococcal conjugates in which serogroup A ("MenA") conjugates are lyophilised for reconstitution by liquid formulations of conjugates from other serogroups.

Hib and meningococcus are two causes of bacterial meningitis, and it is an object of the invention to provide further and improved vaccine formulations for Hib and meningococcal conjugates.

### DISCLOSURE OF THE INVENTION

According to some embodiments of the invention, a liquid Hib component is used to reconstitute a lyophilised meningococcal component, thereby producing a combined meningitis vaccine.

Thus the invention provides a kit comprising: (i) an aqueous component, comprising a conjugate of a *Haemophilus influenzae* type B capsular saccharide at a concentration in the range of 0.5µg/ml to 50µg/ml; and (ii) a lyophilised component, comprising conjugates of *Neisseria meningitidis* capsular saccharides of meningococcal serogroups A, C, W135 and Y, wherein the quantity of the meningococcal saccharides per serogroup is between 1µg and 20µg. For administration to a patient, the aqueous and lyophilised components are combined, to give a combined liquid vaccine that is suitable for injection.

The invention also provides a method for preparing a combined vaccine, comprising the step of combining (i) an aqueous component, comprising a conjugate of a *Haemophilus influenzae* type B capsular saccharide at a concentration in the range of 0.5µg/ml to 50µg/ml, and (ii) a lyophilised component, comprising conjugates of *Neisseria meningitidis* capsular saccharides of meningococcal serogroups A, C, W135 and Y, wherein the quantity of the meningococcal saccharides per serogroup is between 1µg and 20µg.

The invention also provides a combined vaccine comprising: (i) a conjugate of a *Haemophilus influenzae* type B capsular saccharide at a concentration in the range of 0.5µg/ml to 50µg/ml; and (ii) conjugates of *Neisseria meningitidis* capsular saccharides, prepared by combining an aqueous *H.influenzae* conjugate and lyophilised *N.meningitidis* conjugates, wherein the quantity of the meningococcal saccharides per serogroup is between 1µg and 20µg. The vaccine may include lyophilisation stabilisers (see below).

### The liquid component

Kits and methods of the invention involve the use of an aqueous antigenic component that includes a conjugate of a Hib saccharide. Administration of the Hib conjugate preferably results in an anti-PRP antibody concentration in a patient of ≥0.15µg/ml, and more preferably ≥1µg/ml. These are the standard acceptable response thresholds.

Hib saccharide antigens are well known [e.g. chapter 14 of reference 1] and their preparation is well documented [e.g. references 4 to 13]. The Hib saccharide is conjugated to a carrier protein in order to enhance its immunogenicity, especially in children. The invention may use any suitable Hib conjugate.

The saccharide moiety of the Hib conjugate may be a polysaccharide (e.g. full-length polyribosylribitol phosphate (PRP)), but it is also possible to use oligosaccharides (e.g. MW from ∼1 to ∼5 kDa). Oligosaccharides are conveniently formed by fragmentation of purified PRP *(e.g.* by hydrolysis), which will usually be followed by purification of the fragments of the desired size. Where the composition of the invention includes a conjugated oligosaccharide, the preparation of oligosaccharides should precede conjugation.

The concentration of Hib conjugate in the aqueous component is in the range of 0.5µg/ml to 50µg/ml *e.g.* from 1µg/ml to 20µg/ml, from 12µg/ml to 16µg/ml, *etc.* The concentration may be about 15 µg/ml.

The aqueous Hib component may be unadjuvanted or may include an adjuvant. Where an adjuvant is included, it will typically be an aluminium salt *e.g.* a phosphate salt or a hydroxide salt. Where an adjuvant is included, the Hib component may be adsorbed to an aluminium salt or may be unadsorbed. Adsorption to aluminium phosphate adjuvants has been reported to be advantageous in some circumstances [14], whereas non-adsorption has been reported to be advantageous in other circumstances [2].

Various different Hib conjugates are known. For instance, Table 14-7 of reference 1 gives the characteristics of four different Hib conjugates. These differ by various parameters e.g. carrier protein. The invention can use any suitable carrier protein (see below), such as CRM197 (as in 'HbOC'), tetanus toxoid (as in 'PRP-T') and the outer membrane complex of *N.meningitidis* (as in 'PRP-OMP').

Various aqueous Hib conjugates are commercially available and can be used with the invention. For example, Wyeth's HIBTITER™ product is available in a liquid formulation. HIBTITER™ uses a CRM197 carrier and each 0.5ml dose (supplied in vials) contains 10µg of saccharide in 0.9% sodium chloride, with no adjuvant. Merck's PEDVAXHIB™ product is also available in a liquid formulation. PEDVAXHIB™ uses an OMPC carrier and each 0.5ml dose contains 7.5µg of saccharide with 225µg of aluminium hydroxyphosphate sulfate adjuvant in 0.9% sodium chloride. It is free from both lactose and thimerosal. The ACTHIB™ product is not currently available in liquid form. Another useful Hib conjugate comprises a Hib oligosaccharide covalently linked to CRM₁₉₇ via an adipic acid linker [15,16].

The Hib conjugate may be the only antigenic ingredient in the aqueous component, or there may be one or more further antigens. For instance, the aqueous component may include one or more of: a diphtheria toxoid, a tetanus toxoid, acellular pertussis antigen(s), inactivated poliovirus antigen(s), hepatitis B virus surface antigen, and/or pneumococcal saccharide. Where the aqueous component is adjuvanted and includes a Hib conjugate, a diphtheria toxoid, a tetanus toxoid, acellular pertussis antigens, inactivated poliovirus antigens and hepatitis B virus surface antigen, the HEXAVAC™ product may be used. Where the aqueous component is adjuvanted and includes a Hib conjugate, a diphtheria toxoid, a tetanus toxoid, whole cell pertussis antigens, hepatitis B virus surface antigen, the QUINVAXEM™ product may be used. Where the aqueous component is adjuvanted and includes a Hib conjugate, a diphtheria toxoid, a tetanus toxoid, acellular pertussis antigens and poliovirus antigens, the PEDIACEL™ product may be used.

The commercially-available liquid monovalent Hib conjugates are preferred aqueous components for use with the invention, such as the unadjuvanted HIBTITER™ vaccine. Apparent advantages of avoiding an adjuvant when combining Hib and meningococcal conjugates are mentioned in ref. 2.

### The lyophilised component

Kits and methods of the invention involve the use of a freeze-dried antigenic component that includes conjugates of capsular saccharides of meningococcal serogroups A, C, W135 and Y. Administration of the meningococcal conjugate preferably results in a bactericidal antibody response, with an increase in serum bactericidal assay (SBA) titre for the relevant serogroup of at least 4-fold, and preferably at least 8-fold, measured with human complement [17]. If rabbit complement is used to measure SBA titres then the titre increase is preferably at least 128-fold.

Conjugated monovalent vaccines against serogroup C have been approved for human use, and include MENJUGATE™ [18], MENINGITEC™ and NEISVAC-C™. Mixtures of conjugates from serogroups A+C are known [19,20] and mixtures of conjugates from serogroups A+C+W135+Y have been reported [21-24] and were approved in 2005 as the aqueous MENACTRA™ product. The lyophilised component used with the invention includes meningococcal conjugates A+C+W135+Y.

The conjugates may be present at substantially equal masses e.g. the mass of each serogroup's saccharide is within ±10% of each other. A typical quantity per serogroup in the lyophilised component is between 1µg and 20µg *e.g.* between 2 and 10 µg per serogroup, or about 4µg. As an alternative to a substantially equal ratio, a double mass of serogroup A saccharide may be used.

The capsular saccharide of serogroup A meningococcus is a homopolymer of (α1→6)-linked *N*-acetyl-D-mannosamine-1-phosphate, with partial O-acetylation in the C3 and C4 positions. Acetylation at the C-3 position can be 70-95%. Conditions used to purify the saccharide can result in de-O-acetylation (*e.g*. under basic conditions), but it is useful to retain OAc at this C-3 position. In some embodiments, at least 50% *(e.g.* at least 60%, 70%, 80%, 90%, 95% or more) of the mannosamine residues in a serogroup A saccharides are O-acetylated at the C-3 position. Acetyl groups can be replaced with blocking groups to prevent hydrolysis [25], and such modified saccharides are still serogroup A saccharides within the meaning of the invention.

The serogroup C capsular saccharide is a homopolymer of (α 2→9)-linked sialic acid (*N*-acetyl neuraminic acid, or 'NeuNAc'). The saccharide structure is written as →9)-Neu p NAc 7/8 OAc-(α2→ . Most serogroup C strains have O-acetyl groups at C-7 and/or C-8 of the sialic acid residues, but about 15% of clinical isolates lack these O-acetyl groups [26,27].The presence or absence of OAc groups generates unique epitopes, and the specificity of antibody binding to the saccharide may affect its bactericidal activity against O-acetylated (OAc-) and de-O-acetylated (OAc+) strains [28-30]. Serogroup C saccharides used with the invention may be prepared from either OAc+ or OAc-strains. Licensed MenC conjugate vaccines include both OAc- (NEISVAC-C™) and OAc+ (MENJUGATE™ & MENINGITEC™) saccharides. In some embodiments, strains for production of serogroup C conjugates are OAc+ strains, *e.g.* of serotype 16, serosubtype P1.7a,1, *etc..* Thus C:16:P1.7a,1 OAc+ strains may be used. OAc+ strains in serosubtype P1.1 are also useful, such as the C11 strain.

The serogroup W135 saccharide is a polymer of sialic acid-galactose disaccharide units. Like the serogroup C saccharide, it has variable O-acetylation, but at sialic acid 7 and 9 positions [31]. The structure is written as: →4)-D-Neu*p*5Ac(7/90Ac)-α-(2→6)-D-Gal-α-(1→.

The serogroup Y saccharide is similar to the serogroup W135 saccharide, except that the disaccharide repeating unit includes glucose instead of galactose. Like serogroup W135, it has variable O-acetylation at sialic acid 7 and 9 positions [31]. The serogroup Y structure is written as: →4)-D-Neu*p*5Ac(7/90Ac)-α-(2→6)-D-Glc-α-(1→.

The saccharides used according to the invention may be O-acetylated as described above (e.g. with the same O-acetylation pattern as seen in native capsular saccharides), or they may be partially or totally de-O-acetylated at one or more positions of the saccharide rings, or they may be hyper-O-acetylated relative to the native capsular saccharides.

The saccharide moieties in conjugates may comprise full-length saccharides as prepared from meningococci, and/or may comprise fragments of full-length saccharides *i.e.* the saccharides may be shorter than the native capsular saccharides seen in bacteria. The saccharides may thus be depolymerised, with depolymerisation occurring during or after saccharide purification but before conjugation. Depolymerisation reduces the chain length of the saccharides. One depolymerisation method involves the use of hydrogen peroxide [21]. Hydrogen peroxide is added to a saccharide (*e.g.* to give a final H₂O₂ concentration of 1%), and the mixture is then incubated (*e.g.* at about 55°C) until a desired chain length reduction has been achieved. Another depolymerisation method involves acid hydrolysis [22]. Other depolymerisation methods are known in the art. The saccharides used to prepare conjugates for use according to the invention may be obtainable by any of these depolymerisation methods. Depolymerisation can be used in order to provide an optimum chain length for immunogenicity and/or to reduce chain length for physical manageability of the saccharides. In some embodiments, saccharides have the following range of average degrees of polymerisation (Dp): A=10-20; C=12-22; W135=15-25; Y=15-25. In terms of molecular weight, rather than Dp, useful ranges are, for all serogroups: <100kDa; 5kDa-75kDa; 7kDa-50kDa; 8kDa-35kDa; 12kDa-25kDa; 15kDa-22kDa.

In some embodiments, the average molecular weight for saccharides from each of meningococcal serogroups A, C, W135 and Y may be more than 50kDa e.g. ≥75kDa, ≥100kDa, ≥110kDa, ≥120kDa, ≥130kDa, *etc.* [32], and even up to 1500kDa, in particular as determined by MALLS. For instance: a MenA saccharide may be in the range 50-500kDa *e.g.*60-80kDa; a MenC saccharide may be in the range 100-210kDa; a MenW135 saccharide may be in the range 60-190kDa *e.g.*120-140kDa; and/or a MenY saccharide may be in the range 60-190kDa *e.g.* 150-160kDa.

Useful carrier proteins (see below) include CRM197, diphtheria toxoid and/or tetanus toxoid. Where the lyophilised component includes conjugates from more than one meningococcal serogroup then the various conjugates may use different carrier proteins (*e.g.* one serogroup on CRM197, another on tetanus toxoid) or they may use the same carrier protein (*e.g.* saccharides from two serogroups separately conjugated to CRM197 and then combined).

For stability reasons, a lyophilised component may include a stabiliser such as lactose, sucrose and mannitol, as well as mixtures thereof *e.g.* lactose/sucrose mixtures, sucrose/mannitol mixtures, *etc.* The final vaccine may thus contain lactose and/or sucrose. Using a sucrose/mannitol mixture can speed up the drying process. A lyophilised component may also include sodium chloride. Soluble components in the lyophilised material will be retained in the composition after reconstitution.

The lyophilised component may or may not include an adjuvant, such as an aluminium salt.

The lyophilised component preferably does not include a Hib saccharide.

### Packaging compositions of the invention

The wet and dry components used with the invention must be kept separate from each other prior to use. Thus they are packaged separately in the form of a kit. The kit can take various forms.

In some embodiments, the two components are packaged into separate containers. In other embodiments, the two components are packaged into separate chambers of a single container *e.g.* into separate containers of a multi-chamber syringe. A dual-chamber syringe allows two individual compositions to be kept separately during storage, but to be mixed as the syringe plunger is activated.

Lyophilised material will usually be presented in a sealed vial. The vial will have an opening (*e.g.* a rubber seal, a breakable neck, *etc.*) that can maintain sterility while permitting removal of its contents and/or introduction of aqueous material for reconstitution. Vials can be made of various materials *e.g.* of a glass, of a plastic, *etc.*

Aqueous material may also be presented in a vial, but as an alternative may be presented in *e.g.* a syringe. Again, the container will be able to maintain sterility while permitting removal of its contents. A syringe may be applied with or without a needle attached to it; in the latter case, a separate needle may be packaged with the syringe for assembly and use, and the syringe will generally have a tip cap to seal the tip prior to attachment of a needle. Safety needles are preferred. 1-inch 23-gauge, 1-inch 25-gauge and 5/8-inch 25-gauge needles are typical. The plunger in a syringe may have a stopper to prevent the plunger from being accidentally removed during aspiration. Syringes can be made of various materials *e.g.* of a glass, of a plastic, *etc.*

A vial can have a cap *(e.g.* a Luer lock) adapted such that a pre-filled syringe can be inserted into the cap, the contents of the syringe can be expelled into the vial (to reconstitute lyophilised material therein), and the contents of the vial can be removed back into the syringe. After removal of the syringe from the vial, a needle can then be attached and the composition can be administered to a patient. The cap may be located inside a seal or cover, such that the seal or cover has to be removed before the cap can be accessed.

Where material is packaged in a container, the container will usually be sterilized before the material is added to it.

Where a glass container (*e.g*. a syringe or a vial) is used, then it can usefully be made from a borosilicate glass rather than from a soda lime glass.

### Reconstitution

Prior to administration to a patient, the invention involves reconstitution of a freeze-dried antigenic component (containing the meningococcal conjugates) with an aqueous component (containing the Hib conjugate). Reconstitution can involve various steps.

If the components are present in a multi-chamber syringe then actuation of the syringe will combine the aqueous and dried materials. Where the components are present in separate containers, different mixing processes can be used. In some embodiments, aqueous material in a vial can be extracted into a syringe (*e.g.* via a needle), or may already be present in a syringe. The aqueous material can then be transferred from the syringe into a vial containing the lyophilised material (*e.g.* via a needle, which may be the same as or different from a needle previously used to extract aqueous material from a vial). The lyophilised material is thereby reconstituted and can be withdrawn (*e.g.* via a needle, again being the same as or different from a previously-used needle) into a syringe (*e.g.* the same as or different from a previously-used syringe), from which it can be injected into a patient (*e.g*. via a needle, again being the same as or different from a previously-used needle).

Once the lyophilised material and aqueous material have been combined and are present in a delivery device (typically a syringe) then the composition can be administered to a patient. Reconstitution will typically take place immediately prior to administration to a patient *e.g.* no more than 30 minutes prior to injection.

After reconstitution, a composition for administration to a patient will include Hib and meningococcal conjugates. A Hib conjugate originates from original aqueous material and a meningococcal conjugate originates from original lyophilised material.

The mass of Hib saccharide in the reconstituted vaccine of the invention is in the range of 0.5µg to 50µg e.g. from 1-20µg, from 10-15µg, from 12-16µg, etc. The amount may be about 12.5µg. A mass of less than 5µg may be suitable [33] *e.g.* in the range 1-5µg, 2-4µg, or about 2.5µg.

The mass of meningococcal saccharide per serogroup in the reconstituted vaccine is between 1µg and 20µg *e.g.* between 2 and 10 µg per serogroup, or about 5µg or about 10µg. The conjugates may be present at substantially equal masses e.g. the mass of each serogroup's saccharide is within +10% of each other. As an alternative to an equal ratio, a double mass of serogroup A saccharide may be used. Thus a vaccine may include MenA saccharide at 10µg and MenC, W135 and Y saccharides at 5µg each.

In some embodiments, the mass of Hib saccharide will be substantially the same as the mass of a particular meningococcal serogroup saccharide. In some embodiments, the mass of Hib saccharide will be more than *(e.g.* at least 1.5x) the mass of a particular meningococcal serogroup saccharide. In some embodiments, the mass of Hib saccharide will be less than *(e.g.* at least 1.5x) the mass of a particular meningococcal serogroup saccharide.

Where a composition includes saccharide from more than one meningococcal serogroup, there is a mean saccharide mass per serogroup. If substantially equal masses of each serogroup are used then the mean mass will be the same as each individual mass; where non-equal masses are used then the mean will differ *e.g*. with a 10:5:5:5 µg amount for a MenACWY mixture, the mean mass is 6.25µg per serogroup. In some embodiments, the mass of Hib saccharide will be substantially the same as the mean mass of meningococcal saccharide per serogroup. In some embodiments, the mass of Hib saccharide will be more than *(e.g.* at least 1.5x) the mean mass of meningococcal saccharide per serogroup. In some embodiments, the mass of Hib saccharide will be less than *(e.g.* at least 1.5x) the mean mass of meningococcal saccharide per serogroup [34].

### Methods of treatment and Administration of the vaccine

The invention involves the co-administration of Hib and meningococcal conjugates in the form of a combination vaccine. The reconstituted compositions are suitable for administration to human patients, and the invention provides a method of raising an immune response in a patient, comprising the step of administering to the patient a composition of the invention.

The invention also provides a composition of the invention for use in medicine.

The invention also provides the use of (i) an aqueous component, comprising a conjugate of a *Haemophilus influenzae* type B capsular saccharide at a concentration in the range of 0.5µg/ml to 50µg/ml, and (ii) a lyophilised component, comprising conjugates of *Neisseria meningitidis* capsular saccharides of meningococcal serogroups A, C, W135 and Y, wherein the quantity of the meningococcal saccharides per serogroup is between 1µg and 20µg, in the manufacture of a medicament for administration to a patient.

The invention also provides a combination of (i) an aqueous component, comprising a conjugate of a *Haemophilus influenzae* type B capsular saccharide at a concentration in the range of 0.5µg/ml to 50µg/ml, and (ii) a lyophilised component, comprising conjugates of a *Neisseria meningitidis* capsular saccharides of meningococcal serogroups A, C, W135 and Y, wherein the quantity of the meningococcal saccharides per serogroup is between 1µg and 20µg, for use in immunisation.

Reconstituted compositions of the invention are preferably vaccines, for use in the reduction or prevention of bacterial meningitis, including meningococcal meningitis and Hib meningitis.

Patients for receiving the compositions of the invention may be less than 2 years old *e.g.* aged between 0-12 months. One particular group of patients is aged between 1 and 3 months, and has not previously received a meningococcal conjugate vaccine.

In order to have full efficacy, a typical primary immunization schedule for a child may involve administering more than one dose. For example, doses may be at: 0, 2 and 4 months (time 0 being the first dose); 0, 1 and 2 months; 0 and 2 months; 0, 2 and 8 months; *etc.* The first dose (time 0) may be administered at about 2 months of age, or sometimes (*e.g.* in a 0-2-8 month schedule) at around 3 months of age.

Compositions can also be used as booster doses *e.g.* for children, in the second year of life.

Compositions of the invention can be administered by intramuscular injection e.g. into the arm, leg or buttock.

Where compositions of the invention include an aluminium-based adjuvant, settling of components may occur during storage. Aqueous compositions should therefore be shaken before and after reconstitution, prior to administration to a patient.

### Conjugation

The invention uses Hib and meningococcal conjugates in which capsular saccharides are conjugated to carrier proteins. Useful carrier proteins for covalent conjugation are bacterial toxins or toxoids, such as diphtheria toxoid or tetanus toxoid, or derivatives thereof such as the CRM197 diphtheria toxin mutant [35-37]. Other suitable carrier proteins include the *N.meningitidis* outer membrane protein [38], synthetic peptides [39,40], heat shock proteins [41,42], pertussis proteins [43,44], cytokines [45], lymphokines [45], hormones [45], growth factors [45], artificial proteins comprising multiple human CD4⁺ T cell epitopes from various pathogen-derived antigens [46] such as N19 [47], protein D from *H.influenzae* [48-50], pneumolysin [51], pneumococcal surface protein PspA [52], iron-uptake proteins [53], toxin A or B from *C.difficile* [54], *etc.*

Diphtheria toxoid (Dt), tetanus toxoid (Tt) and CRM197 are the main carriers currently in use in pediatric vaccines *e.g.* the Hib conjugates from GSK use Tt as the carrier, the HIBTITER™ product uses CRM197, the pneumococcal conjugates in PREVENAR™ use CRM197, the MENJUGATE™ and MENINGITEC™ products use CRM197, and NEISVAC-C™ uses Tt.

Conjugates with a saccharide:protein ratio (w/w) of between 1:5 (*i.e.* excess protein) and 5:1 (*i.e.* excess saccharide) maybe used e.g. ratios between 1:2 and 5:1 and ratios between 1:1.25 and 1:2.5. As described in reference 55, different meningococcal serogroup conjugates in a mixture can have different saccharide:protein ratios e.g. one may have a ratio of between 1:2 & 1:5, whereas another has a ratio between 5:1 & 1:1.99.

Conjugates may be used in conjunction with free carrier protein [56]. When a given carrier protein is present in both free and conjugated form in a composition of the invention, the unconjugated form may be no more than 5% of the total amount of the carrier protein in the composition as a whole, and more usually present at less than 2% by weight.

Any suitable conjugation reaction can be used, with any suitable linker where necessary.

The saccharide will typically be activated or functionalised prior to conjugation. Activation may involve, for example, cyanylating reagents such as CDAP (e.g. 1-cyano-4-dimethylamino pyridinium tetrafluoroborate [57, 58, *etc*.]). Other suitable techniques use active esters, carbodiimides, hydrazides, norborane, p-nitrobenzoic acid, N-hydroxysuccinimide, S-NHS, EDC, TSTU; see also the introduction to reference 11).

Linkages via a linker group may be made using any known procedure, for example, the procedures described in references 59 and 60. One type of linkage involves reductive amination of the polysaccharide, coupling the resulting amino group with one end of an adipic acid linker group, and then coupling a protein to the other end of the adipic acid linker group [61,62]. Other linkers include β-propionamido [63], nitrophenyl-ethylamine [64], haloacyl halides [65], glycosidic linkages [66], 6-aminocaproic acid [67], ADH [68], C₄ to C₁₂ moieties [69] *etc.* Carbodiimide condenation can also be used [70]. As an alternative to using a linker, direct linkage can be used. Direct linkages to the protein may comprise oxidation of the polysaccharide followed by reductive amination with the protein, as described in, for example, references 71 and 72.

A process involving the introduction of amino groups into the saccharide (*e.g*. by replacing terminal =O groups with -NH₂) followed by derivatisation with an adipic diester (*e.g*. adipic acid N-hydroxysuccinimido diester) and reaction with carrier protein can be used. In another useful reaction, a saccharide is derivatised with a cyanylating reagent, followed by coupling to a protein (direct, or after introduction of a thiol or hydrazide nucleophile group into the carrier), without the need to use a linker. Suitable cyanylating reagents include 1-cyano-4-(dimethylamino)-pyridinium tetrafluoroborate ('CDAP'), p-nitrophenylcyanate and N-cyanotriethylammonium tetrafluoroborate ('CTEA').

As described in reference 73, a mixture can include one conjugate with direct saccharide/protein linkage and another conjugate with linkage via a linker. This arrangement applies particularly when using saccharide conjugates from different meningococcal serogroups e.g. MenA and MenC saccharides may be conjugated via a linker, whereas MenW135 and MenY saccharides may be conjugated directly to a carrier protein.

After conjugation, free and conjugated saccharides can be separated. There are many suitable methods for this separation, including hydrophobic chromatography, tangential ultrafiltration, diafiltration, *etc.* (see also refs. 74 & 75, *etc*.). If a vaccine comprises a given saccharide in both free and conjugated forms, the unconjugated form is usefully no more than 20% by weight of the total amount of that saccharide in the composition as a whole (e.g. ≤15%, ≤10%, ≤5%, ≤2%, ≤1%).

The amount of carrier (conjugated and unconjugated) from each conjugate may be no more than 100µg/ml *e*.*g.* <30µg/ml of carrier protein from each conjugate. Some compositions include a total concentration of carrier of less than 500µg/ml e.g. <400µg/ml, <300µg/ml, <200µg/ml, <100µg/ml, <50µg/ml, *etc.*

### Characteristics of compositions of the invention

In addition to the antigenic components described above, compositions of the invention (both before and after mixing) will generally include a non-antigenic component. The non-antigenic component can include carriers, adjuvants, excipients, buffers, *etc.,* as described in more detail below.

Compositions of the invention will usually include one or more pharmaceutical carrier(s) and/or excipient(s). Sterile pyrogen-free, phosphate-buffered physiologic saline is a typical carrier. A thorough discussion of pharmaceutically acceptable excipients is available in reference 76.

To control tonicity, it is useful to include a physiological salt, such as a sodium salt. Sodium chloride (NaCl) is one such salt, which may be present at between 1 and 20 mg/ml.

Aqueous compositions (before and/or after reconstitution of lyophilised material) will generally have an osmolality of between 200 mOsm/kg and 400 mOsm/kg *e.g.* between 240-360 mOsm/kg, or within the range of 290-320 mOsm/kg.

Compositions of the invention may include one or more buffers. Typical buffers include: a phosphate buffer; a Tris buffer; a borate buffer; a succinate buffer; a histidine buffer; or a citrate buffer. Buffers will typically be included in the 5-20mM range. Such buffers may be included in the aqueous and/or lyophilised components.

The pH of an aqueous composition will generally be between 5.0 and 7.5, and more typically between 5.0 and 6.0 for optimum stability, or between 6.0 and 7.0.

Compositions of the invention are preferably sterile.

Compositions of the invention are preferably non-pyrogenic e.g. containing <1 EU (endotoxin unit, a standard measure) per dose, and preferably <0.1 EU per dose.

Compositions of the invention may be gluten free.

Some vaccines of the invention are unadjuvanted. Other vaccines of the invention include adjuvant. Unadjuvanted vaccines can be made my combining unadjuvanted components. Adjuvanted vaccines can be made by combining multiple adjuvanted components, by combining adjuvanted and unadjuvanted components, or by combining unadjuvanted components with an adjuvant.

Where antigens are adsorbed, a composition may be a suspension with a cloudy appearance. This appearance means that microbial contamination is not readily visible, and so the vaccine may contain a preservative. This is particularly important when the vaccine is packaged in multidose containers. Useful preservatives for inclusion are 2-phenoxyethanol and thimerosal. It is recommended, however, not to use mercurial preservatives (*e.g*. thimerosal) where possible. It is preferred that compositions of the invention contain less than about 25 ng/ml mercury. Such preservatives may be included in the aqueous and/or lyophilised components.

The concentration of any aluminium salts in a composition, expressed in terms of Al³⁺, is preferably less than 5 mg/ml *e.g.* ≤4 mg/ml, ≤3 mg/ml, ≤2 mg/ml, ≤1 mg/ml, <0.85mg/ml, etc.

Compositions of the invention may be administered to patients in 0.5ml doses. References to 0.5ml doses will be understood to include normal variance e.g. 0.5ml±0.05ml.

### Adjuvants

Compositions of the invention may include an adjuvant, and this adjuvant may comprise one or more aluminium salts, and particularly an aluminium phosphate adjuvant and/or an aluminium hydroxide adjuvant. Antigenic components used to prepare compositions of the invention may include aluminium adjuvants before being used *i.e.* they are 'pre-mixed' or 'pre-adsorbed' to the adjuvant(s). Aluminium adjuvants currently in use are typically referred to either as "aluminium hydroxide" or as "aluminium phosphate" adjuvants. These are names of convenience, however, as neither is a precise description of the actual chemical compound which is present (*e.g.* see chapter 9 of reference 77). The invention can use any of the "hydroxide" or "phosphate" salts that are in general use as adjuvants.

The adjuvants known as "aluminium hydroxide" are typically aluminium oxyhydroxide salts, which are usually at least partially crystalline. Aluminium oxyhydroxide, which can be represented by the formula AlO(OH), can be distinguished from other aluminium compounds, such as aluminium hydroxide Al(OH)₃, by infrared (IR) spectroscopy, in particular by the presence of an adsorption band at 1070cm⁻¹ and a strong shoulder at 3090-3100cm⁻¹ (chapter 9 of ref. 77).

The adjuvants known as "aluminium phosphate" are typically aluminium hydroxyphosphates, often also containing a small amount of sulfate. They may be obtained by precipitation, and the reaction conditions and concentrations during precipitation can influence the degree of substitution of phosphate for hydroxyl in the salt. Hydroxyphosphates generally have a PO₄/Al molar ratio between 0.3 and 0.99. Hydroxyphosphates can be distinguished from strict AlPO₄ by the presence of hydroxyl groups. For example, an IR spectrum band at 3164cm⁻¹ (*e.g.* when heated to 200°C) indicates the presence of structural hydroxyls (chapter 9 of ref. 77).

The PO₄/Al³⁺ molar ratio of an aluminium phosphate adjuvant will generally be between 0.3 and 1.2, preferably between 0.8 and 1.2, and more preferably 0.95±0.1. The aluminium phosphate will generally be amorphous, particularly for hydroxyphosphate salts. A typical adjuvant is amorphous aluminium hydroxyphosphate with PO₄/Al molar ratio between 0.84 and 0.92, included at 0.6mg Al³⁺/ml. The aluminium phosphate will generally be particulate. Typical diameters of the particles are in the range 0.5-20µm (*e.g*. about 5-10µm) after any antigen adsorption.

The PZC of aluminium phosphate is inversely related to the degree of substitution of phosphate for hydroxyl, and this degree of substitution can vary depending on reaction conditions and concentration of reactants used for preparing the salt by precipitation. PZC is also altered by changing the concentration of free phosphate ions in solution (more phosphate = more acidic PZC) or by adding a buffer such as a histidine buffer (makes PZC more basic). Aluminium phosphates used according to the invention will generally have a PZC of between 4.0 and 7.0, more preferably between 5.0 and 6.5 *e.g.* about 5.7.

An aluminium phosphate solution used to prepare a composition of the invention may contain a buffer *(e.g.* a phosphate or a histidine or a Tris buffer), but this is not always necessary. The aluminium phosphate solution is preferably sterile and pyrogen-free. The aluminium phosphate solution may include free aqueous phosphate ions *e.g.* present at a concentration between 1.0 and 20 mM, preferably between 5 and 15 mM, and more preferably about 10 mM. The aluminium phosphate solution may also comprise sodium chloride. The concentration of sodium chloride is preferably in the range of 0.1 to 100 mg/ml (*e.g.* 0.5-50 mg/ml, 1-20 mg/ml, 2-10 mg/ml) and is more preferably about 3±1 mg/ml. The presence of NaCl facilitates the correct measurement of pH prior to adsorption of antigens.

### Further antigens that may be included

As well as including conjugated *N.meningitidis* and Hib saccharides, compositions may include one or more further antigens. For instance, they may include antigens from other pathogens, particularly from bacteria and/or viruses. Suitable further antigens may be selected from:
- a diphtheria toxoid ('D')
- a tetanus toxoid ('T')
- a pertussis antigen ('P'), which is typically acellular ('aP')
- a hepatitis B virus (HBV) surface antigen ('HBsAg')
- inactivated poliovirus vaccine (IPV)
- a hepatitis A virus (HAV) antigen
- a capsular saccharide from *Streptococus pneumoniae.*

These antigens will usually originate from the aqueous component of the invention.

### Diphtheria toxoid

*Corynebacterium diphtheria* causes diphtheria. Diphtheria toxin can be treated *(e.g.* using formalin or formaldehyde) to remove toxicity while retaining the ability to induce specific anti-toxin antibodies after injection. These diphtheria toxoids are used in diphtheria vaccines, and are disclosed in more detail in chapter 13 of reference 1. Preferred diphtheria toxoids are those prepared by formaldehyde treatment. The diphtheria toxoid can be obtained by growing *C*.*diphtheriae* in growth medium (e.g. Fenton medium, or Linggoud & Fenton medium), which may be supplemented with bovine extract, followed by formaldehyde treatment, ultrafiltration and precipitation. The toxoided material may then be treated by a process comprising sterile filtration and/or dialysis.

Quantities of diphtheria toxoid can be expressed in international units (IU). For example, the NIBSC supplies the 'Diphtheria Toxoid Adsorbed Third International Standard 1999' [78,79], which contains 160 IU per ampoule. As an alternative to the IU system, the 'Lf' unit ("flocculating units" or the "limes flocculating dose") is defined as the amount of toxoid which, when mixed with one International Unit of antitoxin, produces an optimally flocculating mixture [80]. For example, the NIBSC supplies 'Diphtheria Toxoid, Plain' [81], which contains 300 LF per ampoule, and also supplies 'The 1st International Reference Reagent For Diphtheria Toxoid For Flocculation Test' [82] which contains 900 LF per ampoule.

Compositions typically include between 20 and 80 Lf of diphtheria toxoid, typically about 50 Lf.

By IU measurements, compositions will typically include at least 30IU/dose.

The diphtheria toxoid is usefully adsorbed onto an aluminium hydroxide adjuvant.

### Tetanus toxoid

*Clostridium tetani* causes tetanus. Tetanus toxin can be treated to give a protective toxoid. The toxoids are used in tetanus vaccines, and are disclosed in more detail in chapter 27 of reference 1. Preferred tetanus toxoids are those prepared by formaldehyde treatment. The tetanus toxoid can be obtained by growing *C.tetani* in growth medium *(e.g.* a Latham medium derived from bovine casein), followed by formaldehyde treatment, ultrafiltration and precipitation. The material may then be treated by a process comprising sterile filtration and/or dialysis.

Quantities of tetanus toxoid can be expressed in international units (IU). For example, the NIBSC supplies the 'Tetanus Toxoid Adsorbed Third International Standard 2000' [83,84], which contains 469 IU per ampoule. As an alternative to the IU system, the 'Lf' unit ("flocculating units" or the "limes flocculating dose") is defined as the amount of toxoid which, when mixed with one International Unit of antitoxin, produces an optimally flocculating mixture [80]. For example, the NIBSC supplies 'The 1st International Reference Reagent for Tetanus Toxoid For Flocculation Test' [85] which contains 1000 LF per ampoule.

Compositions will typically include between 5 and 50 Lf of diphtheria toxoid, typically about 20 Lf.

By IU measurements, compositions will typically include at least 40IU/dose.

The tetanus toxoid maybe adsorbed onto an aluminium hydroxide adjuvant, but this is not necessary (*e.g*. adsorption of between 0-10% of the total tetanus toxoid can be used).

### Pertussis antigens

*Bordetella pertussis* causes whooping cough. Pertussis antigens in vaccines are either cellular (whole cell, in the form of inactivated *B.pertussis* cells) or acellular. Preparation of cellular pertussis antigens is well documented [*e.g.* see chapter 21 of ref.1]*e.g.* it may be obtained by heat inactivation of phase I culture of *B.pertussis.* As an alternative, however, acellular antigens can be used.

Where acellular antigens are used, it is preferred to use one, two or (preferably) three of the following antigens: (1) detoxified pertussis toxin (pertussis toxoid, or 'PT'); (2) filamentous hemagglutinin ('FHA'); (3) pertactin (also known as the '69 kiloDalton outer membrane protein'). These three antigens are preferably prepared by isolation from *B.pertussis* culture grown in modified Stainer-Scholte liquid medium. PT and FHA can be isolated from the fermentation broth (e.g. by adsorption on hydroxyapatite gel), whereas pertactin can be extracted from the cells by heat treatment and flocculation (e.g. using barium chloride). The antigens can be purified in successive chromatographic and/or precipitation steps. PT and FHA can be purified by, for example, hydrophobic chromatography, affinity chromatography and size exclusion chromatography. Pertactin can be purified by, for example, ion exchange chromatography, hydrophobic chromatography and size exclusion chromatography. FHA and pertactin may be treated with formaldehyde prior to use according to the invention. PT may be detoxified by treatment with formaldehyde and/or glutaraldehyde. As an alternative to this chemical detoxification procedure the PT may be a mutant PT in which enzymatic activity has been reduced by mutagenesis [86], but detoxification by chemical treatment is more usual.

Acellular pertussis antigens may be adsorbed onto one or more aluminium salt adjuvants. As an alternative, they may be added in an unadsorbed state. Where pertactin is added then it is preferably already adsorbed onto an aluminum hydroxide adjuvant. PT and FHA may be adsorbed onto an aluminum hydroxide adjuvant or an aluminum phosphate. Adsorption of all of PT, FHA and pertactin to aluminum hydroxide is useful.

Compositions will typically include: 1-50 µg/dose PT; 1-50 µg/dose FHA; and 1-50 µg pertactin.

As well as PT, FHA and pertactin, it is possible to include fimbriae *(e.g.* agglutinogens 2 and 3) in an acellular pertussis vaccine.

### Hepatitis B virus surface antigen

Hepatitis B virus (HBV) is one of the known agents that cause viral hepatitis. The HBV virion consists of an inner core surrounded by an outer protein coat or capsid, and the viral core contains the viral DNA genome. The major component of the capsid is a protein known as HBV surface antigen or, more commonly, 'HBsAg', which is typically a 226-amino acid polypeptide with a molecular weight of ∼24 kDa. All existing hepatitis B vaccines contain HBsAg, and when this antigen is administered to a normal vaccinee it stimulates the production of anti-HBsAg antibodies which protect against HBV infection.

For vaccine manufacture, HBsAg has been made in two ways. The first method involves purifying the antigen in particulate form from the plasma of chronic hepatitis B carrier, as large quantities of HBsAg are synthesized in the liver and released into the blood stream during an HBV infection. The second way involves expressing the protein by recombinant DNA methods. HBsAg for use with the method of the invention is preferably recombinantly expressed in yeast cells. Suitable yeasts include, for example, *Saccharomyces* (such as *S.cerevisiae*) or *Hanensula* (such as *H.polymorpha*) hosts.

The HBsAg is usually non-glycosylated. Unlike native HBsAg (*i.e.* as in the plasma-purified product), yeast-expressed HBsAg is generally non-glycosylated, and this is the most preferred form of HBsAg for use with the invention, because it is highly immunogenic and can be prepared without the risk of blood product contamination.

The HBsAg will generally be in the form of substantially-spherical particles (average diameter of about 20nm), including a lipid matrix comprising phospholipids. Yeast-expressed HBsAg particles may include phosphatidylinositol, which is not found in natural HBV virions. The particles may also include a non-toxic amount of LPS in order to stimulate the immune system [87]. HBsAg may be in the form of particles including a lipid matrix comprising phospholipids, phosphatidylinositol and polysorbate 20.

All known HBV subtypes contain the common determinant 'a'. Combined with other determinants and subdeterminants, nine subtypes have been identified: ayw1, ayw2, ayw3, ayw4, ayr, adw2, adw4, adrq- and adrq+. Besides these subtypes, other variants have emerged, such as HBV mutants that have been detected in immunised individuals ("escape mutants"). The usual HBV subtype with the invention is subtype adw2.

In addition to the 'S' sequence, a surface antigen may include all or part of a pre-S sequence, such as all or part of a pre-S 1 and/or pre-S2 sequence.

Quantities of HBsAg are typically expressed in micrograms, and a typical amount of HBsAg per vaccine dose is between 5 and 5 µg *e.g.* 10µg/dose.

Although HBsAg may be adsorbed to an aluminium hydroxide adjuvant in the final vaccine (as in the well-known *ENGERIX-B™* product), or may remain unadsorbed, it will generally be adsorbed to an aluminium phosphate adjuvant [88].

### Inactivated poliovirus vaccine

Poliovirus causes poliomyelitis. Rather than use oral poliovirus vaccine, the invention may use IPV, as disclosed in more detail in chapter 24 of reference 1.

Polioviruses may be grown in cell culture, and a preferred culture uses a Vero cell line, derived from monkey kidney. Vero cells can conveniently be cultured on microcarriers. After growth, virions may be purified using techniques such as ultrafiltration, diafiltration, and chromatography. Prior to administration to patients, polioviruses must be inactivated, and this can be achieved by treatment with formaldehyde.

Poliomyelitis can be caused by one of three types of poliovirus. The three types are similar and cause identical symptoms, but they are antigenically very different and infection by one type does not protect against infection by others. It is therefore preferred to use three poliovirus antigens in the invention: poliovirus Type 1 (*e.g.* Mahoney strain), poliovirus Type 2 *(e.g.* MEF-1 strain), and poliovirus Type 3 (*e.g.* Saukett strain). The viruses are preferably grown, purified and inactivated individually, and are then combined to give a bulk trivalent mixture for use with the invention.

Quantities of IPV are typically expressed in the 'DU' unit (the "D-antigen unit" [89]). It is usual to include between 1-100 DU per viral type per dose *e.g.* about 80 DU of Type 1 poliovirus, about 16 DU of type 2 poliovirus, and about 64 DU of type 3 poliovirus.

Poliovirus antigens are preferably not adsorbed to any aluminium salt adjuvant before being used to make compositions of the invention, but they may become adsorbed onto aluminum adjuvant(s) in the vaccine composition during storage.

### Hepatitis A virus antigens

Hepatitis A virus (HAV) is one of the known agents which causes viral hepatitis. HAV vaccines are disclosed in chapter 15 of reference 1. A useful HAV component is based on inactivated virus, and inactivation can be achieved by formalin treatment. Virus can be grown on human embryonic lung diploid fibroblasts, such as MRC-5 cells. A useful HAV strain is HM175, although CR326F can also be used. The cells can be grown under conditions that permit viral growth. The cells are lysed, and the resulting suspension can be purified by ultrafiltration and gel permeation chromatography.

The amount of HAV antigen, measured in EU (Elisa Units), is typically at least about 500EU/ml.

### Pneumococcal saccharides

Conjugated pneumococcal antigens comprise capsular saccharide antigens from *Streptococcus pneumoniae* conjugated to carrier proteins [*e.g.* refs. 90 to 92]. It is normal to include saccharides from more than one serotype of *S.pneumoniae:* mixtures of polysaccharides from 23 different serotype are widely used, as are conjugate vaccines with polysaccharides from between 5 and 11 different serotypes [93]. For example, PREVNAR™ [94] contains antigens from seven serotypes (4, 6B, 9V, 14, 18C, 19F, and 23F) with each saccharide individually conjugated to CRM197 by reductive amination, with 2µg of each saccharide per 0.5ml dose (4µg of serotype 6B).

Compositions of the invention may include saccharide antigens for at least serotypes 6B, 14, 19F and 23F. Further serotypes may be selected from: 1, 3, 4, 5, 7F, 9V and 18C. 7-valent (as in PREVNAR™), 9-valent *(e.g.* the 7 serotypes from PREVNAR, plus 1 & 5), 10-valent *(e.g.* the 7 serotypes from PREVNAR, plus 1, 5 & 7F) and 11-valent (e.g. the 7 serotypes from PREVNAR, plus 1, 3, 5 & 7F) coverage of pneumococcal serotypes is particularly useful.

Typically, a composition will include between 1µg and 20µg (measured as saccharide) per dose of each serotype that is present.

### General

The term "comprising" encompasses "including" as well as "consisting" *e.g.* a composition "comprising" X may consist exclusively of X or may include something additional *e.g.* X + Y.

The word "substantially" does not exclude "completely" *e.g.* a composition which is "substantially free" from Y may be completely free from Y. Where necessary, the word "substantially" may be omitted from the definition of the invention.

The term "about" in relation to a numerical value x means, for example, *x*±10%.

Unless specifically stated, a process comprising a step of mixing two or more components does not require any specific order of mixing. Thus components can be mixed in any order. Where there are three components then two components can be combined with each other, and then the combination may be combined with the third component, *etc.*

Concentrations of conjugates are defined herein in terms of mass of saccharide, in order to avoid variation due to choice of carrier.

Where an antigen is described as being "adsorbed" to an adjuvant, it is preferred that at least 50% (by weight) of that antigen is adsorbed *e.g.* 50%, 60%, 70%, 80%, 90%, 95%, 98% or more. It is preferred that diphtheria toxoid and tetanus toxoid are both totally adsorbed *i.e.* none is detectable in supernatant. Total adsorption of HBsAg is also preferred.

Where animal (and particularly bovine) materials are used in the culture of cells, they should be obtained from sources that are free from transmissible spongiform encaphalopathies (TSEs), and in particular free from bovine spongiform encephalopathy (BSE).

### MODES FOR CARRYING OUT THE INVENTION

Meningococcal saccharide conjugates have been prepared as described in reference 22. Conjugates from each of serogroups A, C, W135 and Y have been combined without adjuvant to give a tetravalent meningococcal conjugate mixture. This mixture can be freeze-dried in the presence of a stabiliser, such as a sucrose/mannitol mixture, to give a lyophilised meningococcal component that can be reconstituted when required in the future. Lyophilised material is filled into vials, in an amount that will provide 5µg of each saccharide (10µg for serogroup A) after reconstitution.

HIBTITER™ vaccine (Wyeth) and Liquid PEDVAXHIB™ are purchased. The HIBTITER™ vaccine is unadjuvanted, whereas Liquid PEDVAXHIB™ contains an aluminium salt adjuvant. Both vaccines are supplied in glass vials.

The contents of a glass vial are extracted into a syringe through a needle and are injected into a vial containing lyophilised material. After gentle mixing, the contents are removed via a needle into a new syringe. The syringe's needle is replaced with an injection needle and the reconstituted vaccine is injected into a test subject.

In initial animal studies, eight groups of ten CD1 mice are used. Group 1 receives a MenACWY conjugate vaccine prepared by mixing lyophilised MenA conjugate with a liquid MenCWY mixture, as described in reference 22. Group 2 receives a MenACWY vaccine prepared by aqueous reconstitution of the lyophilised MenACWY. Groups 3 to 5 receive the same vaccine as group 2, but reconstituted with: (3) a Hib-OMPC conjugate with aluminium hydroxyphosphate sulfate adjuvant *e.g.* PEDVAXHIB™; (4) a CRM197-Hib conjugate adsorbed to aluminium phosphate; or (5) an unadsorbed CRM197-Hib conjugate *e.g.* HIBTITER™. These three liquid Hib vaccines are administered without meningococcal conjugates to groups 6 to 8 to match groups 3 to 5, respectively. Vaccines are administered subcutaneously in 200µl volumes at days 0, 14 and 28. Doses are 1µg of MenCWY saccharide, 2µg MenA saccharide and 2.5µg Hib saccharide. Sera taken at days 0, 28 and 42 are tested for bactericidal activity against meningococcal strains and for anti-PRP responses. In variants of these experiments, group 4 receives a CRM197-Hib conjugate that is adjuvanted with but not adsorbed to aluminium phosphate.

In study SS-07-02 immunogenicity results after three doses were as follows, expressed as % responders for the five conjugates and also, for the meningococcal conjugates, as geometric mean titers (GMTs):

| | **MenA** | | **MenC** | | **MenW135** | | **MenY** | | **Hib** |
|---|---|---|---|---|---|---|---|---|---|
| | **GMT** | **%** | **GMT** | **%** | **GMT** | **%** | **GMT** | **%** | **%** |
| 1 | 272 | 100 | 154 | 100 | 146 | 100 | 111 | 100 | - |
| 2 | 656 | 100 | 239 | 100 | 106 | 100 | 233 | 100 | - |
| 3 | 1882 | 100 | 1533 | 100 | 1380 | 100 | 530 | 100 | 37.5 |
| 4 | 116 | 87.5 | 205 | 100 | 231 | 100 | 91 | 87.5 | 87.5 |
| 5 | 111 | 100 | 70 | 87.5 | 66 | 75 | 38 | 75 | 75 |
| 6 | - | - | - | - | - | - | - | - | 50 |
| 7 | - | - | - | - | - | - | - | - | 62.5 |
| 8 | - | - | - | - | - | - | - | - | 50 |

Thus the combination of liquid Hib with lyophilised MenACWY can give a composition that retains efficacy against all five bacteria. Good results are seen in group 3 (reconstitution with a liquid Hib conjugate with an aluminium hydroxyphosphate sulfate adjuvant) and group 4 (reconstitution with a liquid Hib conjugate with an aluminium phosphate adjuvant). Group 3 gave the highest anti-meningococcal titres with 100% responders against all serogroups, but had only a modest number of responders to the Hib antigen. The best anti-Hib response was seen in group 4, and anti-meningococcal responses were also reasonable, particularly against serogroup W135 [3].

Groups 3 and 4 also showed good immune responses after two doses (data not shown), and anti-MenY responses in particular were higher in these two groups than in groups 1, 2 or 5 (both in terms of GMTs and % responders). Group 4 showed the highest anti-meningococcal titres after 2 doses with 100% responders against all serogroups.

In groups 4 and 5 the presence of the meningococcal conjugates increased immune responses against the Hib saccharide (compare group 8 with group 5, and group 7 with group 4). In groups 3 and 4, where the liquid Hib antigen was adjuvanted, the presence of the Hib conjugate enhanced anti-W135 titres (compare to group 2), but this enhancement was not seen in group 5, using unadjuvanted Hib.

In parallel experiments, conjugates were reconstituted using a squalene-in-water emulsion (MF59). Three groups (9 to 11) received: (9) lyophilised MenACWY conjugates, (10) a CRM197-Hib conjugate or (11) a mixture of the two. Immunogenicity results after 3 doses were as follows:

| | **MenA** | | **MenC** | | **MenW135** | | **MenY** | | **Hib** |
|---|---|---|---|---|---|---|---|---|---|
| | **GMT** | **%** | **GMT** | **%** | **GMT** | **%** | **GMT** | **%** | **%** |
| 2 & 8 | 656 | 100 | 239 | 100 | 106 | 100 | 233 | 100 | 50 |
| 9 | 1011 | 100 | 501 | 100 | 423 | 100 | 501 | 100 | - |
| 10 | - | - | - | - | - | - | - | - | 87.5 |
| 11 | 1470 | 100 | 600 | 100 | 640 | 100 | 374 | 100 | 100 |

Thus the squalene-in-water emulsion improves efficacy of the meningococcal and Hib conjugates, with the combination of MenACWY+Hib+emulsion offering excellent immunogenicity.

Stability studies on the MenA and Hib saccharide conjugates in combined products 4 and 5 were also performed using NMR over a 24 hour period at room temperature. Both of these saccharides contain phosphodiester bonds and so are susceptible to hydrolytic breakdown during storage in aqueous form. After reconstitution of the lyophilised MenACWY mixture with liquid Hib, however, both the MenA and Hib saccharides remained fully stable in the presence or absence of an aluminium phosphate adjuvant, with no detectable depolymerisation.

It will be understood that the invention has been described by way of example only, and that modifications may be made whilst remaining within the scope of the invention as claimed.

### REFERENCES

*[1]* Vaccines. (eds. Plotkin & Orenstein). 4th edition, 2004, ISBN: 0-7216-9688-0.
[2] WO02/00249.
[3] WO2005/032583.
[4] Ramsay et al. (2001) Lancet 357(9251):195-196.
[5] Lindberg (1999) Vaccines 17 Suppl 2:S28-36.
[6] Buttery & Moxon (2000) JR Coll Physicians Lond 34:163-168.
[7] Ahmad & Chapnick (1999) Infect Dis Clin North Am 13:113-133, vii.
[8] Goldblatt (1998) J. Med. Microbiol. 47:563-567.
[9] European patent 0477508.
[10] US patent 5,306,492.
[11] WO98/42721.
*[12]* Conjugate Vaccines (eds. Cruse et al.) ISBN 3805549326, particularly vol. 10:48-114.
[13] Hermanson (1996) Bioconjugate Techniques ISBN: 0123423368 or 012342335X.
[14] WO97/00697.
[15] Kanra et al. (1999) The Turkish Journal of Paediatrics 42:421-427.
[16] Ravenscroft et al. (2000) Dev Biol (basel) 103: 35-47.
[17] W.H.O. Tech. Rep. Ser. 594:51, 1976.
[18] Jones (2001) Curr Opin Investig Drugs 2:47-49.
[19] Costantino et al. (1992) Vaccine 10:691-8.
[20] Lieberman et al. (1996) JAMA 275:1499-503.
[21] WO02/058737.
[22] WO03/007985.
[23] Rennels et al. (2002) Pediatr Infect Dis J 21:978-979.
[24] Campbell et al. (2002) J Infect Dis 186:1848-1851.
[25] WO03/080678.
[26] Glode et al. (1979) J Infect Dis 139:52-56
[27] WO94/05325; US patent 5,425,946.
[28] Arakere & Frasch (1991) Infect. Immun. 59:4349-4356.
[29] Michon et al. (2000) Dev. Biol. 103:151-160.
[30] Rubinstein & Stein (1998) J. Immunol. 141:4357-4362.
[31] WO2005/033148
[32] WO2007/000314.
[33] WO2007/000327.
[34] WO2007/000322.
[35] Anonymous (Jan 2002) Research Disclosure, 453077.
[36] Anderson (1983) Infect Immun 39(1):233-238.
[37] Anderson et al. (1985) J Clin Invest 76(1):52-59.
[38] EP-A-0372501.
[39] EP-A-0378881.
[40] EP-A-0427347.
[41] WO93/17712
[42] WO94/03208.
[43] WO98/58668.
[44] EP-A-0471177.
[45] WO91/01146
[46] Falugi et al. (2001) Eur J Immunol 31:3816-3824.
[47] Baraldo et al. (2004) Infect Immun 72(8):4884-7.
[48] EP-A-0594610.
[49] Ruan et al. (1990) J Immunol 145:3379-3384.
[50] WO00/56360.
[51] Kuo et al. (1995) Infect Immun 63:2706-13.
[52] WO02/091998.
[53] WO01/72337
[54] WO00/6176.
[55] WO2007/000341.
[56] WO96/40242
[57] Lees et al. (1996) Vaccine 14:190-198.
[58] WO95/08348.
[59] US patent 4,882,317
[60] US patent 4,695,624
[61] Porro et al. (1985) Mo/ Immunol 22:907-919.
[62] EP-A-0208375
[63] WO00/10599
[64] Gever et al. Med. Microbiol. Immunol, 165 : 171-288 (1979).
[65] US patent 4,057,685.
[66] US patents 4,673,574; 4,761,283; 4,808,700.
[67] US patent 4,459,286.
[68] US patent 4,965,338
[69] US patent 4,663,160.
[70] WO2007/000343.
[71] US patent 4,761,283
[72] US patent 4,356,170
[73] WO2007/000342.
[74] Lei et al. (2000) Dev Biol (Basel) 103:259-264.
[75] WO00/38711; US patent 6,146,902.
[76] Gennaro (2000) Remington: The Science and Practice of Pharmacy. 20th edition, ISBN: 0683306472.
*[77]* Vaccine Deign: The Subunit and Adjuvant Approach (eds. Powell & Newman) Plenum Press 1995 (ISBN 0-306-44867-X).
[78] Sesardic et al. (2001) Biologicals 29:107-22.
[79] NIBSC code: 98/560.
[80] Module 1 of WHO's *The immunological basis for immunization series* (Galazka).
[81] NIBSC code: 69/017.
[82] NIBSC code: DIFT.
[83] Sesardic et al. (2002) Biologicals 30:49-68.
[84] NIBSC code: 98/552.
[85] NIBSC code: TEFT.
[86] Rappuoli et al. (1991) TIBTECH9:232-238.
[87] Vanlandschoot et al. (2005) J Gen Virol 86:323-31.
[88] WO93/24148.
[89] Module 6 of WHO's *The immunological basis for immunization series* (Robertson)
[90] Watson (2000) Pediatr Infect Dis J 19:331-332.
[91] Rubin (2000) Pediatr Clin North Am 47:269-285, v.
[92] Jedrzejas (2001) Microbiol Mol Biol Rev 65:187-207.
[93] Zielen et al. (2000) Infect. Immun. 68:1435-1440.
[94] Darkes & Plosker (2002) Paediatr Drugs 4:609-630.
[95] WO99/056776.
[96] US-2007/0014805.
[97] WO2007/080308.
[98] Suli et al. (2004) Vaccine 22(25-26):3464-9.
[99] Allison & Byars (1992) Res Immunol 143:519-25.
[100] Hariharan et al. (1995) Cancer Res 55:3486-9.
[101] WO95/11700.
[102] US patent 6,080,725.
[103] WO2005/097181.
[104] WO2006/113373.

## Claims

1. A kit comprising: (i) an aqueous component, comprising a conjugate of a *Haemophilus influenzae* type B capsular saccharide at a concentration in the range of 0.5µg/ml to 50µg/ml; and (ii) a lyophilised component, comprising conjugates of *Neisseria meningitidis* capsular saccharides of meningococcal serogroups A, C, W135 and Y, wherein the quantity of the meningococcal saccharides per serogroup is between 1µg and 20µg.

2. A method for preparing a combined vaccine, comprising the step of combining (i) an aqueous component, comprising a conjugate of a *Haemophilus influenzae* type B capsular saccharide at a concentration in the range of 0.5µg/ml to 50µg/ml, and (ii) a lyophilised component, comprising conjugates of *Neisseria meningitidis* capsular saccharides of meningococcal serogroups A, C, W135 and Y, wherein the quantity of the meningococcal saccharides per serogroup is between 1µg and 20µg.

3. A combined vaccine comprising: (i) a conjugate of a *Haemophilus influenzae* type B capsular saccharide at a concentration in the range of 0.5µg/ml to 50µg/ml; and (ii) conjugates of *Neisseria meningitidis* capsular saccharides of meningococcal serogroups A, C, W135 and Y, prepared by combining an aqueous *H.influenzae* conjugate and lyophilised *N.meningitidis* conjugates, wherein the quantity of the meningococcal saccharides per serogroup is between 1µg and 20µg.

4. The kit, method or vaccine of any preceding claim, wherein the aqueous component includes an adjuvant.

5. The kit, method or vaccine of any preceding claim, wherein the *H.influenzae* conjugate is adsorbed to aluminium phosphate.

6. The kit, method or vaccine of any one of claims 1 to 3, wherein the aqueous component is unadjuvanted.

7. The kit, method or vaccine of any preceding claim, wherein administration of the *H.influenzae* conjugate results in an anti-PRP antibody concentration in a patient of >0.15µg/ml.

8. The kit, method or vaccine of any preceding claim, wherein the *H.influenzae* saccharide is conjugated to a carrier protein selected from the group consisting of CRM197, tetanus toxoid, and the outer membrane complex of *N.meningitidis.*

9. The kit or method of any preceding claim, wherein the aqueous component comprises one or more of: a diphtheria toxoid, a tetanus toxoid, acellular pertussis antigen(s), inactivated poliovirus antigen(s), hepatitis B virus surface antigen, and/or pneumococcal saccharide.

10. The kit, method or vaccine of any preceding claim, wherein administration of the *N.meningitidis* conjugate(s) results in a bactericidal antibody response.

11. The kit, method or vaccine of any preceding claim, wherein the *Haemophilus influenzae* type B capsular saccharide and the *Neisseria meningitidis* capsular saccharides are conjugated to a bacterial toxin or toxoid.

12. The kit, method or vaccine of any preceding claim, wherein the *N.meningitidis* saccharide(s) is/are conjugated to a carrier protein selected from the group consisting of CRM197, diphtheria toxoid and tetanus toxoid.

13. The kit, method or vaccine of any preceding claim, wherein the lyophilised component includes a stabiliser.

14. The kit, method or vaccine of any preceding claim, wherein the lyophilised component includes an adjuvant.

15. The kit, method or vaccine of any one of claims 1 to 13, wherein the lyophilised component includes no adjuvant.

16. The kit, method or vaccine of any preceding claim, wherein the lyophilised component does not include a Hib saccharide.

17. The vaccine of any preceding claim, including one or more buffers.

18. The vaccine of any preceding claim, wherein the vaccine comprises one or more of: a diphtheria toxoid, a tetanus toxoid, acellular pertussis antigen(s), inactivated poliovirus antigen(s), hepatitis B virus surface antigen, and/or pneumococcal saccharide.

19. The vaccine of any preceding claim, for use as a medicament.

## Patentansprüche

1. Kit, umfassend: (i) eine wässrige Komponente, umfassend ein Konjugat eines Kapsel-Saccharids von *Haemophilus influenzae* Typ B in einer Konzentration im Bereich von 0,5 µg/ml bis 50 µg/ml; und (ii) eine lyophilisierte Komponente, umfassend Konjugate von Kapsel-Sacchariden von *Neisseria meningitidis* der Meningokokken-Serogruppen A, C, W135 und Y, wobei die Menge der Meningokokken-Saccharide pro Serogruppe zwischen 1µg und 20µg liegt.

2. Verfahren zur Herstellung eines kombinierten Impfstoffs, umfassend den Schritt des Kombinierens (i) einer wässrigen Komponente, umfassend ein Konjugat eines Kapsel-Saccharids von *Haemophilus influenzae* Typ B in einer Konzentration im Bereich von 0,5µg/ml bis 50µg/ml; und (ii) einer lyophilisierten Komponente, umfassend Konjugate von Kapsel-Sacchariden von *Neisseria meningitidis* der Meningokokken-Serogruppen A, C, W135 und Y, wobei die Menge der Meningokokken-Saccharide pro Serogruppe zwischen 1µg und 20µg liegt.

3. Kombinierter Impfstoff, umfassend: (i) ein Konjugat eines Kapsel-Saccharids von *Haemophilus influenzae* Typ B in einer Konzentration im Bereich von 0,5µg/ml bis 50µg/ml; und (ii) Konjugate von Kapsel-Sacchariden von *Neisseria meningitidis* der Meningokokken-Serogruppen A, C, W135 und Y, der durch die Kombination eines wässrigen *H.influenzae*-Konjugats und eines lyophilisierten *N.meningitidis*-Konjugats hergestellt wird, wobei die Menge der Meningokokken-Saccharide pro Serogruppe zwischen 1µg und 20µg liegt.

4. Kit, Verfahren oder Impfstoff nach einem der vorstehenden Ansprüche, wobei der wässrige Bestandteil ein Adjuvans umfasst.

5. Kit, Verfahren oder Impfstoff nach einem der vorstehenden Ansprüche, wobei das *H.influenzae*-Konjugat an Aluminiumphosphat adsorbiert ist.

6. Kit, Verfahren oder Impfstoff nach einem der Ansprüche 1 bis 3, wobei der wässrige Bestandteil nicht adjuviert ist.

7. Kit, Verfahren oder Impfstoff nach einem der vorstehenden Ansprüche, wobei die Verabreichung des *H.influenzae-*Konjugats in einem Patienten eine anti-PRP-Antikörper-Konzentration von >0,15µg/ml ergibt.

8. Kit, Verfahren oder Impfstoff nach einem der vorstehenden Ansprüche, wobei das *H.influenzae*-Saccharid an ein Träger-Protein konjugiert ist, das ausgewählt ist aus der Gruppe bestehend aus CRM197, Tetanus-Toxoid und dem äußeren Membrankomplex von *N.meningitidis.*

9. Kit oder Verfahren nach einem der vorstehenden Ansprüche, wobei die wässrige Komponente ein oder mehrere der Folgenden umfasst: ein Diphterie-Toxoid, ein Tetanus-Toxoid, azelluläre(s) Pertussis-Antigen(e), inaktivierte(s) Poliovirus-Antigen(e), Oberflächenantigen des Hepatitis B-Virus und/oder ein Pneumokokken-Saccharid.

10. Kit, Verfahren oder Impfstoff nach einem der vorstehenden Ansprüche, wobei die Verabreichung des/der *N.meningitidis*-Konjugats/Konjugate eine bakterielle Antikörperantwort ergibt.

11. Kit, Verfahren oder Impfstoff nach einem der vorstehenden Ansprüche, wobei das Kapsel-Saccharid von *Haemophilus influenzae* Typ B und die Kapsel-Saccharide von *Neisseria meningitidis* an ein bakterielles Toxin oder Toxoid konjugiert sind.

12. Kit, Verfahren oder Impfstoff nach einem der vorstehenden Ansprüche, wobei das/die *N.meningitidis* Saccharid(e) an ein Träger-Protein konjugiert ist/sind, das ausgewählt ist aus der Gruppe bestehend aus CRM197, Diphterie-Toxoid und Tetanus-Toxoid.

13. Kit, Verfahren oder Impfstoff nach einem der vorstehenden Ansprüche, wobei die lyophilisierte Komponente einen Stabilisator umfasst.

14. Kit, Verfahren oder Impfstoff nach einem der vorstehenden Ansprüche, wobei die lyophilisierte Komponente ein Adjuvans umfasst.

15. Kit, Verfahren oder Impfstoff nach einem der Ansprüche 1 bis 13, wobei die lyophilisierte Komponente kein Adjuvans umfasst.

16. Kit, Verfahren oder Impfstoff nach einem der vorstehenden Ansprüche, wobei die lyophilisierte Komponente kein Hib-Saccharid umfasst.

17. Impfstoff nach einem der vorstehenden Ansprüche, der einen oder mehrere Puffer umfasst.

18. Impfstoff nach einem der vorstehenden Ansprüche, wobei der Impfstoff eine oder mehrere der Folgenden umfasst: ein Diphterie-Toxoid, ein Tetanus-Toxoid, azelluläre(s) Pertussis-Antigen(e), inaktivierte(s) Poliovirus-Antigen(e), Oberflächenantigen des Hepatitis B-Virus und/oder ein Pneumokokken-Saccharid.

19. Impfstoff nach einem der vorstehenden Ansprüche zur Verwendung als Medikament.

## Revendications

1. Kit comprenant : (i) un composant aqueux, comprenant un conjugué à base d'un saccharide capsulaire d'*Haemophilus influenzae* de type B à une concentration dans la plage de 0,5 à 50 µg/ml ; et (ii) un composant lyophilisé, comprenant des conjugués à base de saccharides capsulaires de *Neisseria meningitidis* des sérogroupes méningococciques A, C, W135 et Y, dans lequel la quantité de saccharides méningococciques par sérogroupe est comprise entre 1 et 20 µg.

2. Méthode de préparation d'un vaccin combiné, comprenant des étapes consistant à combiner (i) un composant aqueux, comprenant un conjugué à base d'un saccharide capsulaire d'*Haemophilus influenzae* de type B à une concentration dans la plage de 0,5 à 50 µg/ml, et (ii) un composant lyophilisé, comprenant des conjugués à base de saccharides capsulaires de *Neisseria meningitidis* des sérogroupes méningococciques A, C, W135 et Y, dans lequel la quantité de saccharides méningococciques par sérogroupe est comprise entre 1 et 20 µg.

3. Vaccin combiné comprenant : (i) un conjugué à base d'un saccharide capsulaire d'*Haemophilus influenzae* de type B à une concentration dans la plage de 0,5 à 50 µg/ml ; et (ii) des conjugués à base de saccharides capsulaires de *Neisseria meningitidis* des sérogroupes A, C, W135 et Y, préparé par combinaison d'un conjugué aqueux à base d'*H*. *influenzae* et de conjugués lyophilisés à base *N. meningitidis,* dans lequel la quantité de saccharides méningococciques par sérogroupe est comprise entre 1 et 20 µg.

4. Kit, méthode ou vaccin selon l'une quelconque des revendications précédentes, dans lequel le composant aqueux contient un adjuvant.

5. Kit, méthode ou vaccin selon l'une quelconque des revendications précédentes, dans lequel le conjugué à base de *H. influenzae* est adsorbé sur du phosphate d'aluminium.

6. Kit, méthode ou vaccin selon l'une quelconque des revendications 1 à 3, dans lequel le composant aqueux n'est pas adjuvé.

7. Kit, méthode ou vaccin selon l'une quelconque des revendications précédentes, dans lequel l'administration du conjugué à base de *H. influenzae* induit une concentration d'anticorps anti-PRP chez un patient > 0,15 µg/ml.

8. Kit, méthode ou vaccin selon l'une quelconque des revendications précédentes, dans lequel le saccharide de *H*. *influenzae* est conjugué à une protéine porteuse choisie dans le groupe constitué par CRM197, l'anatoxine tétanique, et le complexe de membrane externe de *N. meningitidis.*

9. Kit ou méthode selon l'une quelconque des revendications précédentes, dans lequel le composant aqueux comprend un ou plusieurs des éléments suivants : une anatoxine diphtérique, une anatoxine tétanique, un ou des antigènes pertussiques acellulaires, un ou des antigènes du virus de la polio inactivé, l'antigène de surface du virus de l'hépatite B, et/ou un saccharide pneumococcique.

10. Kit, méthode ou vaccin selon l'une quelconque des revendications précédentes, dans lequel l'administration du ou des conjugués à base de *N. meningitidis* induit une réponse en anticorps bactéricides.

11. Kit, méthode ou vaccin selon l'une quelconque des revendications précédentes, dans lequel le saccharide capsulaire d'*Haemophilus influenzae* de type B et les saccharides capsulaires de *Neisseria meningitidis* sont conjugués à une toxine ou à une anatoxine bactérienne.

12. Kit, méthode ou vaccin selon l'une quelconque des revendications précédentes, dans lequel le(s) saccharide(s) de *N. meningitidis* est/sont conjugué(s) à une protéine porteuse choisie dans le groupe constitué par CRM197, l'anatoxine diphtérique et l'anatoxine tétanique.

13. Kit, méthode ou vaccin selon l'une quelconque des revendications précédentes, dans lequel le composant lyophilisé contient un stabilisant.

14. Kit, méthode ou vaccin selon l'une quelconque des revendications précédentes, dans lequel le composant lyophilisé contient un adjuvant.

15. Kit, méthode ou vaccin selon l'une quelconque des revendications 1 à 13, dans lequel le composant lyophilisé ne contient pas d'adjuvant.

16. Kit, méthode ou vaccin selon l'une quelconque des revendications précédentes, dans lequel le composant lyophilisé ne contient pas de saccharide Hib.

17. Vaccin selon l'une quelconque des revendications précédentes, contenant un ou plusieurs tampons.

18. Vaccin selon l'une quelconque des revendications précédentes, dans lequel le vaccin comprend un ou plusieurs des éléments suivants : une anatoxine diphtérique, une anatoxine tétanique, un ou des antigènes pertussiques acellulaires, un ou des antigènes du virus de la polio inactivés, l'antigène de surface du virus de l'hépatite B, et/ou un saccharide pneumococcique.

19. Vaccin selon l'une quelconque des revendications précédentes, pouvant être utilisé à titre de médicament.
